# EUROPÄISCHE PATENTSCHRIFT

(11) **EP 0 627 626 B1**
(45) Veröffentlichungstag und Bekanntmachung des Hinweises auf die Patenterteilung: **17.01.1996**
(21) Anmeldenummer: 93108953.6
(22) Anmeldetag: 03.06.1993
(51) Int. Cl.: G01N 33/569

(54) **Nachweis von viralen oder bakteriellen Antigenen mittels Durchflusscytometrie**
Detection of viral or bacterial antigens with flow cytometry
Détection d'antigènes viraux ou bactériens avec cytométrie en milieu fluide

(43) Veröffentlichungstag der Anmeldung: 07.12.1994
(73) Patentinhaber: Burkhardt, Bernd, Dr.med., D-63303 Dreieichenhain (DE)
(72) Erfinder: Burkhardt, Bernd, Dr.med., D-63303 Dreieichenhain (DE)
(74) Vertreter: VOSSIUS & PARTNER

(56) Entgegenhaltungen:
- FR-A- 2 638 849
- GB-A- 2 103 362
- US-A- 4 770 992
- ANALYTICAL CHEMISTRY, Band 65, Nr. 7, 01 April 1993, Washington, DC (US); CASTRO et al., Seiten 849-852

## Beschreibung

Die Erfindung betrifft ein Verfahren zum Nachweis viraler oder bakterieller Antigene. Insbesondere betrifft die Erfindung den Nachweis einer HIV ("Human Immuno-deficiency Virus")-Erkrankung. Die Erfindung betrifft ferner einen Kit zur Durchführung dieses Verfahrens.

Biologische Proben, z.B. EDTA-stabilisiertes Vollblut, enthalten eine Vielzahl verschiedener Zellen, wobei einige dieser Zellen von Viren oder Bakterien infiziert sein können. Die Identifizierung dieser Viren oder Bakterien über spezifische Epitope bzw. Antigene erlaubt die Diagnose einer Infektion. Die Mechanismen der Infektion unterscheiden sich je nach ihrer viralen oder bakteriellen Herkunft. So sind Viren nicht in der Lage, selbständig ihre eigene Struktur vermehren zu können. Sie benötigen daher einen Wirtsorganismus, z.B. eine eukaryotische Zelle, um sich vermehren zu können. Eine durch ein Virus infizierte Zelle wird gezwungen, die Virusstruktur zu replizieren. So infiziert z.B. das HIV-Virus bevorzugt immunkompetente Zellen der T-Lymphozyten, z.B. die Subpopulation CD4. Eine Infektion mit diesem Virus führt zu Veränderungen in diesen Zellen, die letztendlich die biologische Funktion der Zellen beeinträchtigen. Eine derartig hervorgerufenen zelluläre Immundefizienz korreliert mit der Abnahme immunkompetenter Zellen, wodurch eine zelluläre Immunantwort auf opportunistische Infektionen entweder reduziert oder gar nicht mehr möglich ist. Das daraus resultierende Krankheitsbild wird als AIDS bezeichnet.
Obwohl nach dem derzeitigen aktuellen wissenschaftlichen Forschungsstand die Entwicklung einer erfolgversprechenden Therapie als eher zweifelhaft erscheint, ist die Diagnose dieser Erkrankung aus medizinischen und ethischen Überlegungen heraus von großer Bedeutung.

Die derzeit üblichen Verfahren zum Nachweis viraler oder bakterieller Erkrankungen, beispielsweise ELISA-Verfahren zum Antikörpernachweis, sind mit einer Vielzahl von Mängeln behaftet. Insbesondere führt der in diesen Tests durchgeführte Antikörper-Nachweis dazu, daß ein positiv infiziertes Individuum erst geraume Zeit nach Infektion, d.h. also erst bei Bildung der auf das infizierende Virus gerichteten Antikörper, als positiv-infiziert identifiziert wird. Dies führt selbstverständlich zu hoher Unsicherheit bei den durch einen Antikörpernachweis gewonnenen Ergebnissen. Weitere Nachteile herkömmlicher Nachweisverfahren für bakterielle und virale Infektionen liegen in der mangelnden Reproduzierbarkeit der Ergebnisse, dem erforderlichen hohen Fähigkeits- und Kenntnisstand des ausführenden Personals sowie dem hohen Finanz-, Material- und Zeitaufwand.

Die FR-A-2 638 849 offenbart ein Verfahren zum Nachweis viraler und bakterieller Antigene in biologischem Material, wobei fluoreszenzmarkierte Kügelchen, die mit ein oder mehreren verschiedenen Antikörpern gegen ein oder mehrere Antigene beschichtet sind, mit dem biologischen Material gemischt werden und der resultierende Komplex mittels Druchflußcytometrie nachgewiesen wird.

Es ist daher Aufgabe der vorliegenden Erfindung, einen Test zum Nachweis bakterieller oder viraler Antigene bzw. Epitope in biologischem Material zur Verfügung zu stellen, der in kurzer Zeit auf einfache Art und Weise zu verlässlichen Ergebnissen führt.
Die Lösung dieser Aufgabe wird durch die in den Patentansprüchen aufgeführten Ausführungsformen bereitgestellt. Insbesondere besteht die Lösung darin, daß ein Verfahren zum Nachweis viraler oder bakterieller Antigene bzw. Epitope in biologischem Material bereitgestellt wird, das dadurch gekennzeichnet ist, daß das Antigen bzw. Epitop mittels Durchflußcytometrie nachgewiesen wird.

Entscheidend ist allein die Fähigkeit des Antigens, mit einem entsprechenden Antikörper einen Komplex bilden zu können. Demgemäß ist das Verfahren sowohl zum Nachweis intrazellulärer als auch extrazellulärer (z.B. im Serum vorkommender Antigene) Antigene verwendbar.

Durchflußcytometrische Verfahren werden derzeit bei einer Reihe spezieller Fragestellungen eingesetzt. Die Einsatzgebiete reichen von rein wissenschaftlicher Forschung bis hin zur Routinediagnostik im Rahmen laboratoriumsmedizinischer Analysen. Beispielhaft seien hier Analysen der Zellzyklen, der Toxizitäts- und Phagocytoseaktivitäten sowie Verfahren zum Nachweis, zur Differenzierung und Quantifizierung von Zellpopulationen (z.B. Immunstaten).

Zum Nachweis und zur Analyse von intra- oder extrazellulären viralen oder bakteriellen Antigenen bzw. Epitopen wurde die Durchflußcytometrie bisher noch nicht eingesetzt. Insbesondere wurde die Durchflußcytometrie noch nicht zum Nachweis des viralen Antigens p24 des HIV-Virus verwendet. Der Nachweis dieses speziellen Antigens dient im Rahmen von HIV-Bestätigungstests (z.B. Western Blotting) als indikativ für eine manifeste HIV-Infektion.

Das erfindungsgemäße Verfahren ist kostengünstig, schnell durchzuführen, sicher und weist eine vergleichsweise hohe Reproduzierbarkeit der Ergebnisse auf. Ein Einsatz in der laboratoriumsmedizinischen Routine-Analyse (z.B. Nachweis von HIV, transfusionsmedizinische Indikation, Blutkonservensicherheit, epidemiologische Fragestellungen, therapeutische Konzepte, etc.) ist wünschenswert und trifft in diagnostischen und prognostischen Bereichen auf breite Anwendungsmöglichkeiten.

Demgemäß betrifft die Erfindung ein Verfahren zum Nachweis intrazellulärer oder extrazellulärer viraler oder bakterieller Antigene in biologischem Material, dadurch gekennzeichnet, daß nach Durchführung einer oder mehrerer Antigen-Antikörper-Reaktionen und/oder einer sekundären Markierung der (des) erhaltenen Antigen-Antikörper-Komplexe(s) mit fluoreszenzmarkierten Antikörpern die (der) fluoreszenzmarkierte(n) Antigen-Antikörper-Komplex(e) mittels Durchflußcytometrie nachgewiesen werden (wird), mit der Maßgabe, daß die Markierung der Antigen-Antikörper-Komplexe nicht unter Einsatz von Kügelchen erfolgt.

Die Erfindung betrifft auch ein Verfahren zum Nachweis intrazellulärer viraler oder bakterieller Antigene bzw. Epitope in biologischen Materialien, insbesondere umfassend die folgenden Schritte:
(a) Lyse der Zellmembranen von viren- und/oder bakterien-haltigen Zellpopulationen, wobei die zu untersuchenden Antigene erhalten bleiben;
(b) Durchführen einer oder mehrerer Antigen-Antikörper-Reaktionen mittels fluoreszenzmarkiertem Antikörper zum Nachweis einer unspezifischen Reaktion;
(c) Durchführen einer oder mehrerer Antigen-Antikörper-Reaktionen zum Nachweis einer spezifischen Reaktion;
(d) Sekundäre Markierung(en) eines oder mehrerer Antigen-Antikörper-Komplexe(s) aus Schritt (c) mittels fluoreszenzmarkiertem Antikörper;
(e) Anreicherung und Reinigung der aus Schritt (d) erhaltenen Antigen-Antikörper-Komplexe; und
(f) Durchflußcytometrische Untersuchung und Analyse der aus Schritt (e) erhaltenen Antigen-Antikörper-Komplexe.

Zur Fluoreszenz-Markierung des in Schritt (d) genannten sekundären Antikörpers können übliche Fluoreszenz-Marker, wie markierte(s) Biotin, Avidin, Peroxidase und insbesondere FITC verwendet werden.

Die Erfindung betrifft außerdem ein vorstehend genanntes Verfahren, das durch Verwendung einer Trypsin-Lösung zur Lyse der Zellmembranen gekennzeichnet ist. Der erfindungsgemäße Nachweis extrazellulärer Antigene wird insbesondere mittels der vorstehend beschriebenen Abfolge von Verfahrensschritten erreicht, wobei jedoch Schritt (a) durch übliche Isolierungs- und Aufarbeitungsschritte der verwendeten biologischen Materialien ersetzt wird.

Insbesondere ist das erfindungsgemäße Verfahren dadurch gekennzeichnet, daß der Nachweis der viralen oder bakteriellen Antigene mittels
(a) konfigurierter Geräte zur durchflußcytometrischen Analyse und/oder
(b) konfigurierter Software zur Auswertung durchflußcytometrischer Ergebnisse und/oder
(c) Messung mit oder ohne definierten "gates",
durchgeführt wird.

Bei der Verwendung von FITC-fluoreszenzmarkierten Antikörpern ist es zweckmäßig, bei der Gerätekonfiguration des im Ausführungsbeispiel erwähnten Durchflußcytometers eine Kompensation von 0,7-1,1 % bei FL1-%FL2, von 19,4-19,8 % bei FL2-%FL1, von 0,0-0,5 % bei FL2-%FL3, von 0,0-0,5 % bei FL3-%FL2 und insbesondere von 0,9 % bei FL1-%FL2, von 19,6 % bei FL2-%FL1, von 0,0 % bei FL2-%FL3 und von 0,0 % bei FL3-%FL2 zu wählen.

In einer besonders bevorzugten Ausführungsform sind die vorstehend genannten Verfahren dadurch gekennzeichnet, daß die nachgewiesenen Antigene in menschlichem biologischem Material nachgewiesen werden. Die Erfindung betrifft ferner eines der vorstehend genannten Verfahren, wobei eine Kombination verschiedener Antikörper zum spezifischen Antigen-Nachweis unter Verwendung verschiedener fluoreszenzmarkierter (z.B. FITC) sekundärer Antikörper zum Nachweis unterschiedlicher viraler oder bakterieller Antigene eingesetzt werden.

Schließlich betrifft die Erfindung ein vorstehend genanntes Verfahren, das zum Nachweis eines Antigens vom HIV-Virus, insbesondere des p24 Antigens, verwendet wird.

Die Erfindung betrifft außerdem einen Kit zur Verwendung in dem erfindungsgemäßen Verfahren zum Nachweis intrazellulärer Antigene bzw. Epitope umfassend eine Trypsin-Lösung, einen markierten Antikörper, einen unmarkierten, für das nachzuweisende Antigen spezifischen Antikörper und, gegebenenfalls, eine Pufferlösung.

Die Erfindung wird durch das folgende Ausführungsbeispiel näher erläutert.

### Ausführungsbeispiel

### 1. Verwendete Materialien und Gerätekonfiguration

A. Durchflußcytometer (z.B. FACSCAN®, Becton & Dickinson)
B. Kompatible Software (z.B. FACSCAN RESEARCH SOFTWARE, Becton & Dickinson, PC-Lysis SOFTWARE, Becton & Dickinson)
C. Trypsin-Lösung (z.B. Solution-A, Cycle TEST DNA® Kit, Nr. 340020, Becton & Dickinson)
D. Gepufferte Lösung (z.B. Cellwash® (Stabilisierter PBS-Puffer), Becton & Dickinson)
E. Antikörper (z.B. Anti-HIV-1-p24, Immunglobulin Subklasse IgG1, Katalog Nr. NEA 9306, Dupont de Nemour), 1:12.5 Verdünnung
F. Antikörper (z.B. Anti-Maus Antikörper aus der Ziege, FITC-Konjugat, Nr. 349031, Becton & Dickinson).

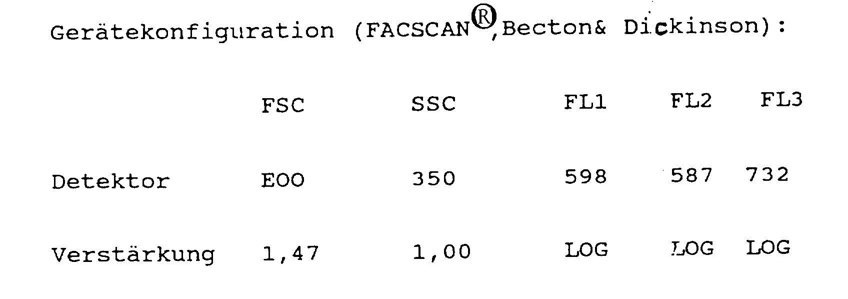

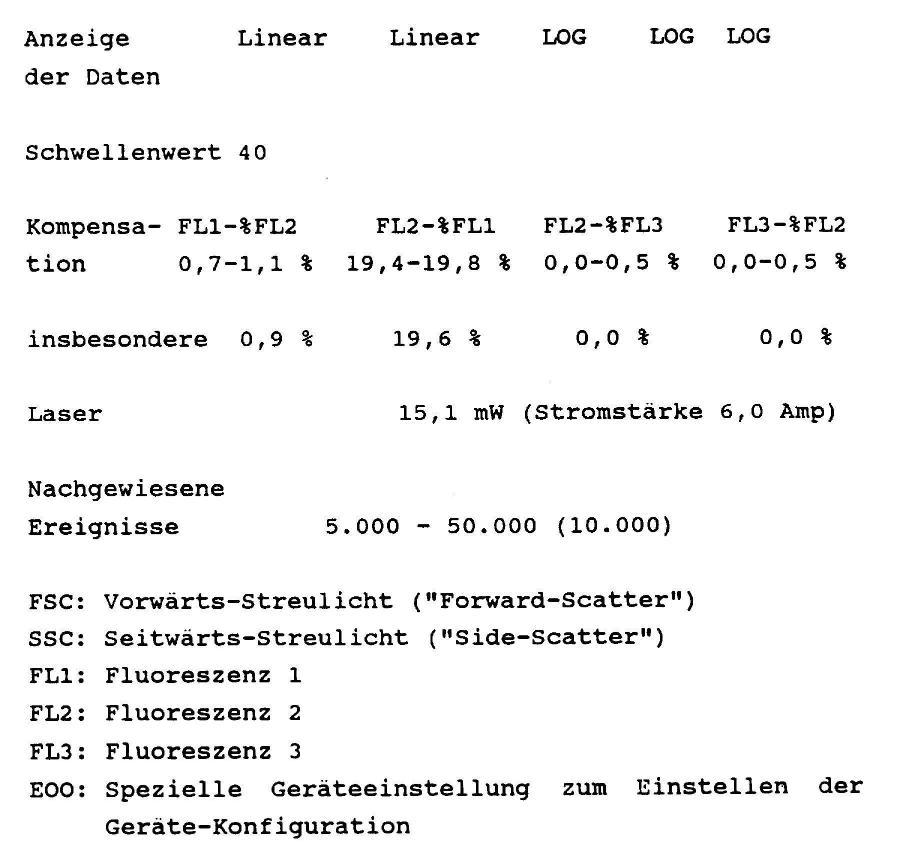

Die Gerätekonfiguration ist abhängig von der Art des verwendeten Geräts, den eingesetzen Materialien und den verwendeten Fluoreszenz-Markern.

### 2. Aufbereitung der Proben

Das erfindungsgemäße Verfahren wird in zwei Ansätzen durchgeführt.
In Gefäß 1 wird als Negativ-Kontrolle die unspezifische Bindung des fluoreszenzmarkierten Anti-Maus-Antikörpers aus der Ziege bestimmt.
In Gefäß 2 wird die spezifische Bindung des Antikörpers Anti-HIV-1-p24 an dessen Antigen durch Bindung mit dem fluoreszenzmarkierten Anti-Maus-Antiköper aus der Ziege nachgewiesen.

### 2.1 Negativ-Kontrolle (unspezifische Bindung, Gefäß 1)

100 µl EDTA-Vollblut werden mit 1000 µl Trypsin-Lösung versetzt, 10 Sekunden bei 1.500 Umdrehungen pro Minute (Vortex) gemischt und 20 bis 40 Minuter, insbesondere 30 Minuten, im Dunkeln inkubiert. Anschließend werden 20 µl fluoreszenzmarkierte Anti-Maus Antikörper aus der Ziege zugegeben, 10 Sekunden bei 1.500 Umdrehungen pro Minute (Vortex) gemischt und 40 Minuten im Dunkeln inkubiert. Die Suspension wird 10 Minuten bei 1.000 x g zentrifugiert, der Überstand dekantiert und der Rückstand nach Zugabe von 500 µl "Cellwash" 10 Sekunden bei 1.500 Umdrehungen pro Minute (Vortex) gemischt. Die erhaltene Suspension wird im Dunkeln 10 Minuten inkubiert.

### 2.2 Antikörper Anti-HIV-1-p24 (spezifische Bindung, Gefäß 2)

100 µl EDTA-Vollblut werden mit 1.000 µl Trypsin-Lösung versetzt, 10 Sekunden bei 1.500 Umdrehungen pro Minute (Vortex) gemischt und 20 bis 40 Minuten, insbesondere 30 Minuten, im Dunkeln inkubiert. Anschließend werden 40 µl Antikörper Anti-HIV-1-p24 zugegeben, 10 Sekunden bei 1.500 Umdrehungen pro Minute (Vortex) gemischt und 20 Minuten im Dunkeln inkubiert. Die Suspension wird mit 20 µl fluoreszenzmarkiertem Anti-Maus-Antikörper aus der Ziege versetzt, 10 Sekunden bei 1.500 Umdrehungen pro Minute (Vortex) gemischt und 20 Minuten im Dunkeln inkubiert. Anschließend wird die Suspension 10 Minuten bei 1.000 x g zentrifugiert, der Überstand dekantiert und der Rückstand mit 500 µl "Cellwash" resuspendiert und 10 Sekunden bei 1.500 Umdrehungen pro Minute (Vortex) gemischt. Die Probe wird anschließend 10 Minuten im Dunkeln inkubiert.

### 3. Durchführung der Messungen

Die Messungen werden an einem Durchflußcytometer (FACSCAN, Becton & Dickinson) im Punktplot-Modus durchgeführt.
Die Durchflußrate kann im Modus "slow" oder bei zu erwartenden Raten von weniger als 200 nachzuweisenden Ereignissen im Modus "fast" erfolgen. Die Anzahl der pro Messung zu erfassenden Ereignisse zeigt Abhängigkeit von Probenvolumen und der Dichte der enthaltenden fluoreszenzmarkierten Antigen-Antikörper-Komplexe an.

### 4. Auswertung

Die Auswertung und Analyse erfolgt im Punktplot- (Fluoreszenz 1 - Fluoreszenz 3) bzw. im Histogramm-Modus (Fluoreszenz 1 - Autocount). Die Definition der "gates" zur Analyse erfolgt im Punktplot-Modus (Fluoreszenz 1 - Fluoreszenz 3). Diese Definition ist optional.
Die Analyse der Messungen erfolgt über eine kompatible Software, beispielsweise FACSCAN RESEARCH SOFTWARE, Becton & Dickinson oder PC-LYSIS SOFTWARE, Becton & Dickinson.

### 4.1 Die Auswertung erfolgt in Fluoreszenz 1 im Histogramm-Verfahren ("Gates Off") :

### 4.1.1 Auswertung der unspezifischen Bindung als Kontrollwert (Gefäß 1)

A) Position der Marker (3) auf Fluoreszenz-Kanäle:
   1.) 512 2.) 768 3.) 1023
B) Berechnung der statistischen Parameter.

### 4.1.2 Auswertung der spezifischen Bindung (Gefäß 2)

A) Position der Marker analog zu 4.1.1 A).
B) Berechnung der statistischen Parameter.

### 4.1.3 Die Auswertung erfolgt anhand der relativen Verteilung der gemessenen Fluoreszenzen. Zur Standardisierung der Auswertung und deren Dokumentation werden die statistischen Daten, entsprechend den Markerpositionen 512, 768 und 1023 berechnet. Eine umfassende Erklärung des durchflußcytometrischen Auswertungsverfahrens ist in "FACSCAN-Research-Software"-Handbuch, Becton & Dickinson, Nr. 11-10285-00 REV.A. (Nov. 1987) gegeben.

### 4.2 Ergebnisse der Auswertung (Tabelle 1)

Die Kontrollen zur unspezifischen Bindung (Gefäß 1) HIV-negativer Proben zeigen von Markerpositionen 512 bis 768 im Mittel einen Nachweisfaktor von 0,71 mit einem Mittelwert bei Kanal 622. Die Messungen zur spezifischen Bindung (Gefäß 2) HIV-negativer Proben zeigen von Markerpositionen 512 bis 768 im Mittel einen Nachweisfaktor von 0,32 mit einem Mittelwert bei Kanal 625 (s. Tab. 1).

Die Kontrollen zur unspezifischen Bindung (Gefäß 1) HIV-positiver Proben zeigen von Markerpositionen 512 bis 768 im Mittel einen Nachweisfaktor von 15,56 mit einem Mittelwert bei Kanal 610. Die Messungen zur spezifischen Bindung (Gefäß 2) HIV-positiver Proben zeigen von Markerpositionen 512 bis 768 im Mittel einen Nachweisfaktor von 29,23 mit einem Mittelwert bei Kanal 667 (s. Tab. 1).

Die Kontrollen zur unspezifischen Bindung (Gefäß 1) HIV-negativer Proben zeigen von Markerpositionen 769 bis 1023 im Mittel einen Nachweisfaktor von 0,013 mit einem Mittelwert bei Kanal 900. Die Messungen zur spezifischen Bindung (Gefäß 2) HIV-negativer Proben zeigen von Markerpositionen 769 bis 1023 im Mittel einen Nachweisfaktor von 0,13 mit einem Mittelwert bei Kanal 903 (s. Tab. 1).

Die Kontrollen zur unspezifischen Bindung (Gefäß 1) HIV-positiver Proben zeigen von Markerpositionen 769 bis 1023 im Mittel einen Nachweisfaktor von 0,51 mit einem Mittelwert bei Kanal 763. Die Messungen zur spezifischen Bindung (Gefäß 2) HIV-positiver Proben zeigen von Markerpositionen 769 bis 1023 im Mittel einen Nachweisfaktor von 25,08 mit einem Mittelwert bei Kanal 888 (s. Tab. 1).

Vergleicht man die Relation der spezifischen Bindung (Gefäß 2) von HIV-negativen Proben mit HIV-positiven Proben für die Markerpositionen 512 bis 768 und 769 bis 1023 wird deutlich, daß das erfindungsgemäße Verfahren einen eindeutigen Nachweis des viralen p24 Antigens erlaubt.

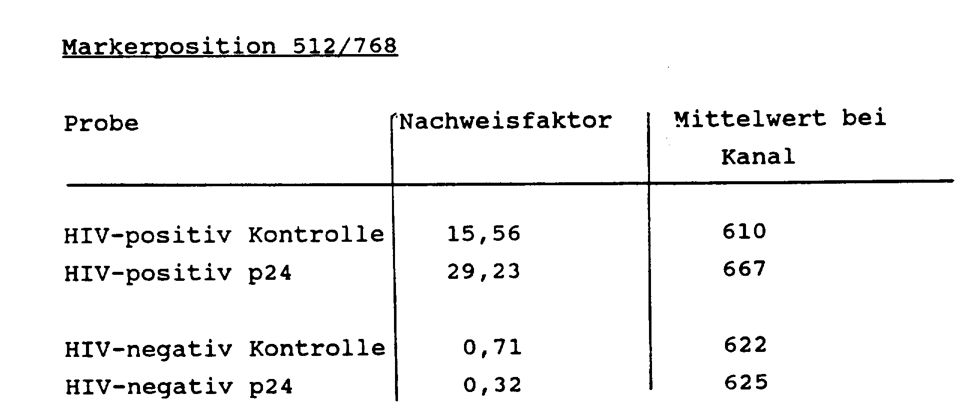

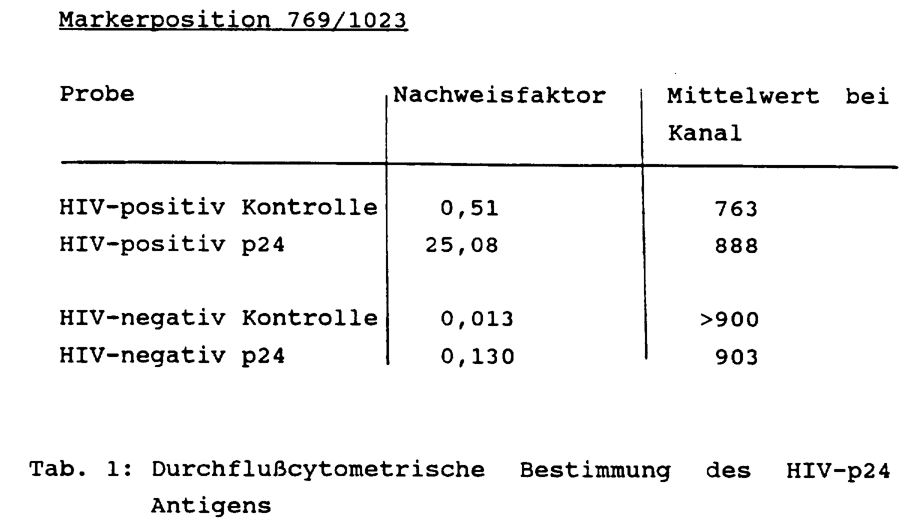

## Patentansprüche

1. Verfahren zum Nachweis intrazellulärer oder extrazellulärer viraler oder bakterieller Antigene in biologischem Material, dadurch gekennzeichnet, daß nach Durchführung einer oder mehrerer Antigen-Antikörper-Reaktionen und/oder einer sekundären Markierung der (des) erhaltenen Antigen-Antikörper-Komplexe(s) mit fluoreszenzmarkierten Antikörpern die (der) fluoreszenzmarkierte(n) Antigen-Antikörper-Komplex(e) mittels Durchflußcytometrie nachgewiesen werden (wird), mit der Maßgabe, daß die Markierung der Antigen-Antikörper-Komplexe nicht unter Einsatz von Kügelchen erfolgt.

2. Verfahren nach Anspruch 1 umfassend die folgenden Schritte:
(a) Lyse der Zellmembranen von viren- und/oder bakterien-haltigen Zellpopulationen, wobei die zu untersuchenden Antigene erhalten bleiben;
(b) Durchführen einer oder mehrerer Antigen-Antikörper-Reaktionen mittels fluoreszenzmarkiertem Antikörper zum Nachweis einer unspezifischen Reaktion;
(c) Durchführen einer oder mehrerer Antigen-Antikörper-Reaktionen zum Nachweis einer spezifischen Reaktion;
(d) Sekundäre Markierung(en) eines oder mehrerer Antigen-Antikörper-Komplexe(s) aus Schritt (c) mittels fluoreszenzmarkiertem Antikörper;
(e) Anreicherung und Reinigung der in Schritt (d) erhaltenen Antigen-Antikörper-Komplexe; und
(f) Durchflußcytometrische Untersuchung und Analyse der in Schritt (e) erhaltenen Antigen-Antikörper-Komplexe.

3. Verfahren nach Anspruch 1 oder 2, dadurch gekennzeichnet, daß zur Lyse der Zellmembranen eine Trypsin-Lösung verwendet wird.

4. Verfahren nach einem der Ansprüchen 1 bis 3, dadurch gekennzeichnet, daß der Nachweis der viralen oder bakteriellen Antigene mittels
(a) konfigurierter Geräte zur durchflußcytometrischen Analyse und/oder
(b) konfigurierter Software zur Auswertung durchflußcytometrischer Ergebnisse und/oder
(c) Messung mit oder ohne definierten "gates"
durchgeführt wird.

5. Verfahren nach Anspruch 4, dadurch gekennzeichnet, daß der fluoreszenzmarkierte Antikörper mit FITC markiert ist.

6. Verfahren nach Anspruch 5, dadurch gekennzeichnet, daß die Kompensation FL3-%FL2 0,0-0,5 % beträgt.

7. Verfahren nach einem der Ansprüche 1 bis 6, dadurch gekennzeichnet, daß das nachgewiesene Antigen in menschlichem biologischem Material nachgewiesen wird.

8. Verfahren nach einem der Ansprüche 1 bis 7, dadurch gekennzeichnet, daß eine Kombination von Antikörpern zum Nachweis verschiedener viraler oder bakterieller Antigene eingesetzt wird.

9. Verfahren nach einem der Ansprüche 1 bis 8, dadurch gekennzeichnet, daß das nachgewiesene Antigen vom HIV-Virus stammt.

10. Verfahren nach einem der Ansprüche 1 bis 8, dadurch gekennzeichnet, daß das nachgewiesene Antigen p24 ist.

11. Kit zur Verwendung in dem Verfahren nach einem der Ansprüche 1 bis 10, umfassend eine Trypsin-Lösung, einen markierten Antikörper, einen unmarkierten, für das nachzuweisende Antigen spezifischen Antikörper und, gegebenenfalls, eine Pufferlösung mit der Maßgabe, daß die Markierung der Antigen-Antikörper-Komplexe nicht unter Einsatz von Kügelchen erfolgt.

## Claims

1. A method for detecting intracellular or extracellular viral or bacterial antigens in biological material, characterized in that after carrying out one or more antigen-antibody reactions and/or a secondary labelling of one or more antigen-antibody complexes with fluorescent labelled antibodies, the one or more fluorescent labelled antigen-antibody complexes are detected by means of flow-cytometry, with the proviso that the labelling of the antigen-antibody complexes does not include the use of beads.

2. The method according to claim 1, comprising the following steps:
(a) lysis of the cell membranes of cell populations containing viruses and/or bacteria while maintaining the antigens to be examined;
(b) carrying out one or more antigen-antibody reactions with a fluorescent labelled antibody for detecting an unspecific reation;
(c) carrying out one or more antigen-antibody reactions for detecting a specific reaction;
(d) secondary fluorescence labelling of one or more antigen-antibody complexes of step (c) via a fluorescent labelled antibody;
(e) enrichment and purification of the antigen-antibody complexes obtained by step (d); and
(f) flow-cytometric examination and analysis of the antigen-antibody complexes obtained by step (e).

3. The method according to claim 1 or 2, characterized in that a trypsin solution is used for the lysis of the cell membranes.

4. The method according to any of claims 1 to 3, characterized in that the viral or bacterial antigens are detected by means of
(a) adjusted instruments of flow-cytometric analysis and/or
(b) configured software for evaluating the results of the flow-cytometric analysis and/or
(c) a measurement with or without defined "gates".

5. The method according to claim 4, characterized in that the fluorescent labelled antibody is labelled with FITC.

6. The method according to claim 5, characterized in that the compensation FL3-%FL2 is 0.0 to 0.5 %.

7. The method according to any of claims 1 to 6, characterized in that the detected antigen is detected in human biological material.

8. The method according to any of claims 1 to 7, characterized in that a combination of antibodies is used for detecting various viral or bacterial antigens.

9. The method according to any of claims 1 to 8, characterized in that the detected antigen stems from the HIV virus.

10. The method according to any of claims 1 to 8, characterized in that the detected antigen is p24.

11. A kit for use in the method according to any of claims 1 to 10, comprising a trypsin solution, a labelled antibody, an unlabelled antibody specific for the antigen to be detected and, optionally, a buffer solution, with the proviso that the labelling of the antigen-antibody complexes does not include the use of beads.

## Revendications

1. Procédé de détection d'antigènes viraux ou bactériens, intracellulaires ou extracellulaires, dans une matière biologique, caractérisé en ce que, après mise en oeuvre d'une ou de plusieurs réactions antigène-anticorps et/ou d'un marquage secondaire du ou des complexes antigène-anticorps obtenus par des anticorps marqués par fluorescence, le ou les complexes antigène-anticorps marqués par fluorescence est ou sont décelés au moyen d'une cytométrie de flux, à la condition que le marquage des complexes antigène-anticorps n'ait pas lieu avec mise en oeuvre de sphérules.

2. Procédé suivant la revendication 1, comprenant les étapes suivantes :
(a) une lyse des membranes cellulaires de populations cellulaires contenant des virus et/ou des bactéries, les antigènes à examiner étant maintenus,
(b) une mise en oeuvre d'une ou de plusieurs réactions antigène-anticorps au moyen d'un anticorps marqué par fluorescence, pour déceler une réaction non spécifique,
(c) une mise en ouvre d'une ou de plusieurs réactions antigène-anticorps, pour déceler une réaction spécifique,
(d) un ou des marquages secondaires d'un ou de plusieurs complexes antigène-anticorps de l'étape (c) au moyen d'un anticorps marqué par fluorescence,
(e) un enrichissement et une purification des complexes antigène-anticorps obtenus dans l'étape (d), et
(f) un examen et une analyse à cytométrie de flux des complexes antigène-anticorps obtenus dans l'étape (e).

3. Procédé suivant l'une des revendications 1 et 2, caractérisé en ce que, pour la lyse des membranes cellulaires, on utilise une solution de trypsine.

4. Procédé suivant l'une des revendications 1 à 3, caractérisé en ce que la détection des antigènes viraux ou bactériens est effectuée au moyen
(a) d'instruments configurés pour l'analyse à cytométrie de flux et/ou
(b) des softwares configurés pour l'évaluation des résultats de cytométrie de flux, et/ou
(c) d'une mesure avec ou sans "portes" (gates) définies.

5. Procédé suivant la revendication 4, caractérisé en ce que l'anticorps marqué par fluorescence est marqué avec du FITC.

6. Procédé suivant la revendication 5, caractérisé en ce que la compensation pour FL3-%FL2 est de 0,0-0,5 %.

7. Procédé suivant l'une des revendications 1 à 6, caractérisé en ce que l'antigène détecté est décelé dans une matière biologique humaine.

8. Procédé suivant l'une des revendications 1 à 7, caractérisé en ce qu'une combinaison d'anticorps est mise en oeuvre pour déceler des antigènes viraux ou bactériens différents.

9. Procédé suivant l'une des revendications 1 à 8, caractérisé en ce que l'antigène décelé est issu du virus VIH.

10. Procédé suivant l'une des revendications 1 à 8, caractérisé en ce que l'antigène décelé est p24.

11. Trousse à utiliser dans le procédé suivant l'une des revendications 1 à 10, comprenant une solution de trypsine, un anticorps marqué, un anticorps non marqué, spécifique pour l'antigène à déceler, et, éventuellement, une solution tampon, avec la condition que le marquage des complexes antigène-anticorps n'ait pas lieu avec mise en oeuvre de sphérules.
